# EUROPEAN PATENT SPECIFICATION

(11) **EP 2 642 974 B1**
(45) Date of publication and mention of the grant of the patent: **19.05.2021**
(21) Application number: 11781527.4
(22) Date of filing: 08.11.2011
(51) Int. Cl.: A61K 8/35, A61K 8/60, A61Q 15/00, A61K 8/67

(54) **PROCESS FOR TREATING PERSPIRATION USING A CARBONYL COMPOUND CAPABLE OF REACTING VIA THE MAILLARD REACTION**
VERFAHREN ZUR BEHANDLUNG VON SCHWEISSSEKRETION MIT EINER ÜBER DIE MAILLARD-REAKTION REAKTIONSFÄHIGEN CARBONYLVERBINDUNG
PROCÉDÉ DE TRAITEMENT DE LA TRANSPIRATION UTILISANT UN COMPOSÉ DE CARBONYLE CAPABLE DE RÉAGIR VIA LA RÉACTION DE MAILLARD

(30) Priority: 25.11.2010 FR 1059729; 29.11.2010 US 417699 P
(43) Date of publication of application: 02.10.2013
(73) Proprietor: L'Oréal, 75008 Paris (FR)
(72) Inventor: BERNARD, Dominique, F-92170 Vanves (FR); AUBRUN, Odile, F-92160 Antony (FR)
(74) Representative: L'Oreal
(86) International application number: PCT/EP2011/069664
(87) International publication number: WO 2012/069310

(56) References cited:
- EP-A1- 2 136 445
- EP-A2- 0 561 489
- DE-A1- 2 401 489
- GB-A- 1 514 216
- GB-A- 2 136 445
- US-A1- 2006 003 969

## Description

The invention relates to the cosmetic use, as an agent for treating perspiration, of at least one compound comprising at least one free carbonyl function that is capable of reacting with at least one amine compound via the Maillard reaction, and more particularly with skin proteins, said at least one compound comprising at least one free carbonyl function being a carbohydrate and/or a precursor thereof and/or an oxidized form thereof, and in which the carbohydrate is dihydroxyacetone (DHA) in free form and/or in encapsulated form.

In the cosmetics field, it is well known to use in topical application, as antiperspirants, aluminium and/or zirconium salts, which have the effect of limiting or even preventing the flow of sweat. These products are generally available in the form of roll-ons, sticks, aerosols or sprays.

Metal salts of this type are efficient as antiperspirant active agents, but some people find that the application of such products causes skin irritation. Furthermore, they also have a tendency to leave marks on clothing.

There is thus still a need to find novel formulations for treating perspiration that do not have the drawbacks encountered with those known hitherto, and which give good antiperspirant efficacy. There is thus a need to find novel antiperspirant active agents that can replace aluminium salts and aluminium/zirconium salts, and that are efficient, easy to formulate and well tolerated.

In patent application JP 05058867, polyols, for instance glycerol, 1,3-butanediol, sorbitol, glucose and maltitol, have already been proposed in combination with a lower alcohol (ethanol) and water in antiperspirant compositions for the purpose of increasing the rate of disappearance of perspiration on open areas of the human body, such as the face.

Patent IT 2006PD034 describes hypoallergenic body deodorant products which may contain from 5% to 20% of a carbohydrate such as fructose.

Patent DE 102008059765 describes deodorants/antiperspirant products using the glycation compounds (combination of aldoses with amino acids) or precursors thereof, for the purpose of inhibiting hair growth.

The Applicant has discovered, surprisingly and unexpectedly, that the use of at least one compound comprising at least one free carbonyl function being a carbohydrate and/or a precursor thereof and/or an oxidized form thereof, and in which the carbohydrate is dihydroxyacetone (DHA) in free form and/or in encapsulated form that is capable of reacting with at least one amine compound via the Maillard reaction, and more particularly with skin proteins, makes it possible to achieve this objective.

This discovery forms the basis of the invention.

A subject of the present invention is the cosmetic use, as an agent for treating perspiration, of at least one compound comprising at least one free carbonyl function that is capable of reacting with at least one amine compound via the Maillard reaction, and more particularly with proteins of the horny layer of the skin, said at least one compound comprising at least one free carbonyl function being a carbohydrate and/or a precursor thereof and/or an oxidized form thereof, and in which the carbohydrate is dihydroxyacetone (DHA) in free form and/or in encapsulated form.

Other subjects of the invention will emerge later in the description.

The term "cosmetically acceptable" means compatible with the skin and/or its integuments or mucous membranes, having a pleasant colour, odour and feel and not causing any unacceptable discomfort (stinging, tautness or redness) liable to discourage the consumer from using this composition.

The term "human keratin materials" means the skin (of the body, face and around the eyes), hair, eyelashes, eyebrows, bodily hair, nails, lips or mucous membranes.

The term "agent for treating perspiration" means any substance which has the effect of reducing the flow of sweat and/or of reducing the sensation of moisture associated with human sweat, and/or of masking human sweat.

The term "compound comprising at least one free carbonyl function" means any simple molecule or polymer comprising at least one free carbonyl function, especially at least one ketone or aldehyde function.

The term "amine compound" means any simple molecule or polymer comprising at least one free amine function in its structure. Mention may be made especially of amino acids, proteins and peptides, and more particularly the proteins of the horny layer of the skin.

### MAILLARD REACTION

The Maillard reaction, discovered by Louis-Camille Maillard, was made public on 27 November 1911 in a communication. This reaction comprises the following main mechanisms:

### 1) Maillard condensation

The carbonyl group of the compound comprising at least one free carbonyl function, generally a sugar, reacts with the amine group of the amine compound, for example an amino acid, producing N-substituted glycosylamines and water.

Since the carbon atom (C) of the carbonyl group of the sugar is electrophilic, and the nitrogen atom (N) of the amino acid is, on the other hand, nucleophilic, they will have a tendency to attract each other. In order for N and C to bond together, the oxygen atom (O) of the carbonyl transforms one of its bonds with the carbon non-bonding lone pair, since O is more electronegative (electron-withdrawing tendency) than C: it then becomes negatively charged since it is electron-rich:

-C=O -> -C-O-

The nitrogen atom of the amino acid then transforms its non-bonding lone pair to bond to the carbon, which cannot remain with only three electrons in the outer shell. The nitrogen atom is then electron-poor (it needs to recover its non-bonding lone pair) and is positively charged. Since it is more electronegative than hydrogen, it will then transform with its hydrogen into a non-bonding lone pair, the hydrogen released bonding with the oxygen that had created a non-bonding lone pair, to enable the amino acid to condense with the sugar. This reversible process is known as phototropism:

OH-G-C-O⁻ + A-NH₂ -> OH-G-OHC-NH-A

The molecule then stabilizes, the alcohol function at the end of the chain becomes detached, and the oxygen transforms its bond with the rest of the molecule into a non-bonding lone pair. This creates a possibility of bonds for the carbon, and it uses the electron released to form a second bond with the nitrogen of the amino acid, which, itself, in order to furnish an electron, breaks a bond with one of its two hydrogens. The OH⁻ ion and the hydrogen ion H⁻ then bond together, creating a water molecule. The remaining molecule is known as a Schiffs base, but is still unstable.

OH-G-OHC-NH-A-> G-OHC=N-A + H₂O

### 2) Amadori or Heyns rearrangement

The glycosylamines undergo an Amadori or Heyns rearrangement to form ketosamines.

In acidic medium, the nitrogen bonds to the H⁺ ions, characteristic of acidic medium, by means of its non-bonding lone pair, thus becoming positively charged. The carbon in the second position then breaks its bond with a hydrogen and thus creates a possibility of bonding with the other carbon, resulting in H⁺ ions. The first carbon atom, which cannot form five bonds, will give an electron to the nitrogen, thus filling its electron deficit (the nitrogen transforms it into a non-bonding lone pair). It is moreover noted that the acidity of the medium is not modified since the H⁺ ions are returned to the medium.

At the first alcohol function, the oxygen gives rise to a release of H⁺ ions by transforming its bond with the H into a lone pair. The carbon in the second position breaks one of its two bonds with the first carbon to re-establish the equilibrium: the first carbon captures the H⁺ ion, and the second bonds with the oxygen, filling its electron deficit. This transformation is known as a keto-enol equilibrium since it transforms an alcohol (enol) function into a ketone (keto) function, and does so in both directions, although the passage from -OH to =O is predominant.

### 3) Moderate dehydrogenation

In the moderate dehydrogenation pathway, the oxygen transforms one of its two bonds with the carbon in the second position into a non-bonding lone pair. Simultaneously, the carbon in the third position on the carbon backbone transforms its bond with a hydrogen into an additional bond with the carbon in the second position. Release then takes place of an electron-poor H⁺ ion, which bonds with the lone pair of the oxygen.

This process is known as enolization, since it creates an alcohol function from a ketone function and a hydrogen atom. This is referred to in this case as 2 (position of the carbon to which the ketone function is bonded), 3 (position of the carbon to which the hydrogen atom is bonded) enolization.

The OH⁻ ions, characteristic of basic medium, will then participate in the reaction as catalyst. The H transforms its bond with the oxygen into a non-bonding lone pair since the oxygen, although electronegative, is already electron-rich. The H⁻ ion released then creates a bond with the nitrogen, which, in order to receive it, separates from the main chain, abandoning the electron of its bond with the carbon.

On the main carbon chain, the double bond between carbons 2 and 3 is transformed into a bond with one of the two oxygens of the alcohol functions, in so doing bringing about a release of H⁻ ions (for the same reasons as previously) which will then bond with the O⁻ ion, thus recreating the OH⁻ ion (the acidity of the medium is thus preserved).

At the alcohol function located on carbon 3, the oxygen transforms its bond with the hydrogen into an additional bond with the carbon, thus stabilizing it. An H⁺ ion will then be released with the C⁻ ion.

A second enolization (3,4) then takes place: the oxygen transforms one of its two bonds with carbon 3 into a non-bonding lone pair. Simultaneously, the carbon in the fourth position on the carbon backbone transforms its bond with the hydrogen into a bond with carbon 3. Release then takes place of an electron-poor H⁺ ion. The oxygen bonded to carbon 3 then transforms its excess lone pair into a bond with the H⁺ ion, which thus overcomes its electron deficiency.

The molecule thus obtained is known as a reductone (it has lost its amino acid and now contains a ketone function).

The carbon in the fifth position transforms its bond with the H into a non-bonding lone pair, releasing an H⁺ ion. The carbon in the third position transforms one of the two bonds with carbon 4 into a bond with the H⁺ ion, and, finally, carbon 5 transforms its non-bonding lone pair into a bond with carbon 4 to stabilize the whole.

Preferentially, the compound(s) comprising at least one free carbonyl function of the invention are present in the compositions in concentrations ranging from 0.1% to 20% by weight and preferably from 1% to 15% by weight relative to the total weight of the composition.

According to one particular embodiment of the invention, the compound(s) comprising at least one free carbonyl function of the invention may be combined with amine compounds, for instance amino acids such as lysine or proline, or with agents that are capable of modulating the conformation of proteins, for instance chaotropic agents (for example urea or urea derivatives, guanidine salts), cosmotropic agents (sulfates, phosphates, lithium, magnesium, tert-butanol), surfactants, dehydrating agents or protease inhibitors.

The two compounds are applied to the skin sequentially (two actions) or simultaneously (extemporaneous preparation, etc.).

### REACTIVE CARBONYL COMPOUNDS

### 1) Carbohydrates

Among the compounds comprising at least one free carbonyl function that is capable of reacting with at least one amine compound via the Maillard reaction, use will be made in particular of a carbohydrate and/or a precursor thereof and/or an oxidized form thereof.

The term "carbohydrate" means any organic molecule containing a carbonyl group (aldehyde or ketone) and several hydroxyl groups (-OH). Carbohydrates were historically known as carbon hydrates. Their chemical formula is based on the model Cₙ(H₂O)ₚ (whence derives the historical name). However, this model is not suitable for all carbohydrates, some of which contain heteroatoms such as nitrogen or phosphorus.

The term "carbohydrate precursor" means any chemical substance that is capable of producing at least one carbohydrate by enzymatic hydrolysis on contact with the epidermis.

Carbohydrates (or carbon hydrates) usually comprise:
(1) monosaccharides or saccharides, which are simple, non-hydrolysable crystal-forming molecules. They are of two types: (a) aldoses comprising an aldehyde function on the first carbon and ketoses comprising a ketone function on the second carbon. They are also distinguished according to the number of carbon atoms they contain.
(2) oligosaccharides or oligosides, which are saccharide polymers bearing a sequence of monosaccharides comprising from 2 to 10 monosaccharide units linked via glycoside bonds.
(3) polyholosides (polysaccharides), which are saccharide polymers bearing a sequence of more than 10 monosaccharide units (for example: amylose, amylopectin, cellulose, glycogen).

Among the oligosides and polyosides, the following are distinguished:
- homopolyosides are carbohydrates whose hydrolysis gives only one type of saccharide;
- heterosides and heteropolyosides are carbohydrates whose hydrolysis does not give only one type of saccharide. These are polymers of saccharides and of non-saccharide molecules. An example of a heteroside that may be mentioned is salicin.

Saccharides, also known as monosaccharides, may be used as agents for treating perspiration.

Among the saccharides or monosaccharides that may be used, mention may be made of:
- trioses containing three carbons: dihydroxyacetone;
- tetroses containing four carbons: erythrose, threose, erythrulose;
- pentoses containing five carbons: ribose, arabinose, xylose, lyxose, ribulose, xylulose, deoxyribose;
- hexoses containing six carbons: allose, altrose, glucose, mannose, fucose, gulose, idose, galactose, talose, fuculose, psicose, fructose, sorbose, tagatose, quinovose, pneumose, rhamnose;
- heptoses containing seven carbons: sedoheptulose, glucoheptose, idoheptulose, mannoheptulose, taloheptulose;
- octoses containing eight carbons;
in their D or L form.

Mention may also be made of oxidized carbohydrates, for instance 2-keto- and 5-keto-D-gluconic acid and D-glucurono-gamma-lactone.

According to the invention, dihydroxyacetone (DHA) will be used as carbohydrate.

The saccharide(s) in accordance with the invention may be used in free and/or encapsulated form, for example in lipid vesicles such as liposomes, especially described in patent application WO 97/25970.

Among the carbohydrate precursors that may be used according to the invention, examples that may be mentioned include:
ester precursors of dihydroxyacetone such as dihydroxyacetone phosphates.

### 2) Other reactive carbonyl compounds

Among the compounds comprising at least one free carbonyl function that is capable of reacting with at least one amine compound via the Maillard reaction, use may be made of vitamin C, derivatives thereof and degradation products thereof, for example:
- via oxidation, such as dehydroascorbic acid, also known as threo-2,3-hexodiulsono-1,4 lactone, 9CI, (CAS 490-83-5) and/or monomeric or polymeric derivatives thereof (especially dimers) as described in patent application EP 2 016 932;
- by dehydration and decarboxylation, for instance α-pyrone, 3-hydroxy-2-pyranone and furfural.

### GALENICAL FORMS

The carbohydrate-based compositions for use in the invention may be in any galenical form conventionally used for topical application and especially in the form of aqueous gels, or aqueous or aqueous-alcoholic solutions. By adding a fatty or oily phase, it may also be in the form of dispersions of lotion type, emulsions of liquid or semi-liquid consistency of milk type, obtained by dispersing a fatty phase in an aqueous phase (O/W) or conversely (W/O), or suspensions or emulsions of soft, semi-solid or solid consistency of the cream or gel type, or alternatively multiple emulsions (W/O/W or O/W/O), microemulsions, vesicular dispersions of ionic and/or nonionic type, or wax/aqueous phase dispersions. These compositions are prepared according to the usual methods.

The compositions for use in the invention may especially be conditioned in pressurized form in an aerosol device or in a pump-action bottle; conditioned in a device equipped with a perforated wall, especially a grille; conditioned in a device equipped with a ball applicator ("roll-on"); conditioned in the form of wands (sticks) or in the form of loose or compacted powder. In this regard, they contain the ingredients generally used in products of this type, which are well known to those skilled in the art.

According to one particular form of the invention, the compositions for use in the invention may be anhydrous.

The term "anhydrous composition" means a composition containing less than 2% by weight of water, or even less than 0.5% water, and especially free of water, the water not being added during the preparation of the composition but corresponding to the residual water provided by the mixed ingredients.

According to another particular form of the invention, the compositions for use in the invention may be solid, in particular in wand or stick form.

The term "solid composition" means that the maximum force measured by texturometry during the penetration of a probe into the sample of formula must be at least equal to 0.25 newtons, in particular at least equal to 0.30 newtons and especially at least equal to 0.35 newtons, assessed under precise measuring conditions as follows.

The formulae are poured hot into jars 4 cm in diameter and 3 cm deep. Cooling is performed at room temperature. The hardness of the formulae is measured after an interval of 24 hours. The jars containing the samples are characterized in texturometry using a texturometer such as the machine sold by the company Rheo TA-XT2, according to the following protocol: a stainless-steel ball probe 5 mm in diameter is brought into contact with the sample at a speed of 1 mm/s. The measuring system detects the interface with the sample, with a detection threshold equal to 0.005 newtons. The probe penetrates 0.3 mm into the sample, at a speed of 0.1 mm/s. The measuring machine records the change in force measured in compression over time, during the penetration phase. The hardness of the sample corresponds to the average of the maximum force values detected during penetration, over at least three measurements.

### AQUEOUS PHASE

According to one particularly preferred form, the compositions for use in the invention comprise an aqueous phase and more preferentially comprise at least 20% by weight of water relative to the total weight of the composition.

They are especially formulated as aqueous lotions or as water-in-oil or oil-in-water emulsions or as multiple emulsions (oil-in-water-in-oil or water-in-oil-in-water triple emulsion (such emulsions are known and described, for example, by C. Fox in "Cosmetics and Toiletries" - November 1986 - Vol. 101 - pages 101-112)).

The aqueous phase of the said compositions contains water and may also contain other water-soluble or water-miscible solvents. The water-soluble or water-miscible solvents especially comprise short-chain monoalcohols, for example of C₁-C₄, for instance ethanol or isopropanol.

### EMULSIFIERS

### a) Oil-in-water emulsifiers

As emulsifiers that may be used in the oil-in-water emulsions or oil-in-water-in-oil triple emulsions, examples that may be mentioned include nonionic emulsifiers such as oxyalkylenated (oxyethylenated and/or oxypropylenated) fatty acid esters; oxyalkylenated (oxyethylenated and/or oxypropylenated) fatty alkyl ethers.

Mention may also be made of fatty alcohol/alkylpolyglycoside emulsifying mixtures as described in patent applications WO 92/06778, WO 95/13863 and WO 98/47610, for instance the commercial products sold by the company SEPPIC under the name Montanov®.

### b) Water-in-oil emulsifiers

Among the emulsifiers that may be used in the water-in-oil emulsions or water-in-oil-in-water-in-oil triple emulsions, examples that may be mentioned include alkyl dimethicone copolyols corresponding to formula (I) below 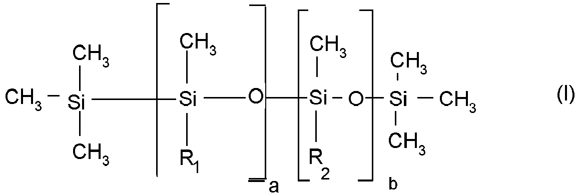 in which:
R₁ denotes a linear or branched C₁₂-C₂₀ and preferably C₁₂-C₁₈ alkyl group;
R₂ denotes the group: -CₙH₂ₙ--(-OC₂H₄-)ₓ--(-OC₃H₆-)_{y}--O-R₃;
R₃ denotes a hydrogen atom or a linear or branched alkyl radical comprising from 1 to 12 carbon atoms;
a is an integer ranging from 1 to 500 approximately;
b is an integer ranging from 1 to 500 approximately;
n is an integer ranging from 2 to 12 and preferably from 2 to 5;
x denotes an integer ranging from 1 to 50 approximately and preferably from 1 to 30;
y denotes an integer ranging from 0 to 49 approximately and preferably from 0 to 29, with the proviso that when y is other than zero, the ratio x/y is greater than 1 and preferably ranges from 2 to 11.

Among the alkyl dimethicone copolyol emulsifiers of formula (I) that are preferred, mention will be made more particularly of Cetyl PEG/PPG-10/1 Dimethicone and more particularly the mixture Cetyl PEG/PPG-10/1 Dimethicone and Dimethicone (INCI name), for instance the product sold under the trade name Abil EM90 by the company Goldschmidt, or alternatively the mixture (Polyglyceryl-4 Stearate and Cetyl PEG/PPG-10 (and) Dimethicone (and) Hexyl Laurate), for instance the product sold under the trade name Abil WE09 by the same company.

Among the water-in-oil emulsifiers, mention may also be made of the dimethicone copolyols corresponding to formula (II) below 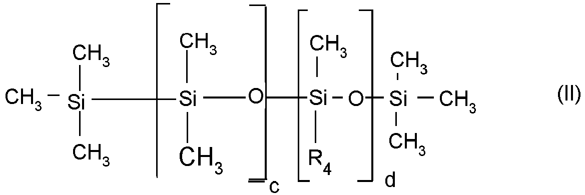 in which
R₄ denotes the group: -CₘH₂ₘ--(-OC₂H₄-)ₛ--(-OC₃H₆-)ₜ--O-R₅,
R₅ denotes a hydrogen atom or a linear or branched alkyl radical comprising from 1 to 12 carbon atoms,
c is an integer ranging from 1 to about 500;
d is an integer ranging from 1 to about 500;
m is an integer ranging from 2 to 12 and preferably from 2 to 5;
s is an integer ranging from 1 to 50 approximately and preferably from 1 to 30;
t is an integer ranging from 0 to 50 approximately and preferably from 0 to 30; with the proviso that the sum s+t is greater than or equal to 1.

Among these preferential dimethicone copolyol emulsifiers of formula (II), use will particularly be made of PEG-18/PPG-18 Dimethicone and more particularly the mixture Cyclopentasiloxane (and) PEG-18/PPG-18 Dimethicone (INCI name), such as the product sold by the company Dow Corning under the trade name Silicone DC5225 C or KF-6040 from the company Shin-Etsu.

According to one particularly preferred form, use will be made of a mixture of at least one emulsifier of formula (I) and of at least one emulsifier of formula (II).

Use will be made more particularly of a mixture of PEG-18/PPG-18 Dimethicone and Cetyl PEG/PPG-10/1 Dimethicone and even more particularly a mixture of (Cyclopentasiloxane (and) PEG-18/PPG-18 Dimethicone) and of Cetyl PEG/PPG-10/1 Dimethicone and Dimethicone or of (Polyglyceryl-4-stearate and Cetyl PEG/PPG-10 (and) Dimethicone (and) Hexyl Laurate).

Among the water-in-oil emulsifiers, mention may also be made of nonionic emulsifiers derived from fatty acids and polyol.

As nonionic emulsifiers derived from fatty acids and polyol, use may be made especially of fatty acid esters of polyols, the fatty acid especially containing a C8-C24 alkyl chain.

Fatty acid esters of polyols that may especially be mentioned include isostearic acid esters of polyols, and stearic acid esters of polyols, and mixtures thereof.

Stearic acid esters of polyols that may especially be mentioned include the polyethylene glycol esters, for instance PEG-30 Dipolyhydroxystearate, such as the product sold under the name Arlacel P135 by the company ICI.

The emulsifier may also be chosen from alkylpolyglycosides with an HLB of less than 7, for example those represented by the general formula (1) below:

R-O-(G)x (1)

in which R represents a branched and/or unsaturated alkyl radical comprising from 14 to 24 carbon atoms, G represents a reduced sugar comprising 5 or 6 carbon atoms, and x is a value ranging from 1 to 10 and preferably from 1 to 4, and G especially denotes glucose, fructose or galactose.

The unsaturated alkyl radical may comprise one or more ethylenic unsaturations, and in particular one or two ethylenic unsaturations.

As alkylpolyglycosides of this type, mention may be made of alkylpolyglucosides (G = glucose in formula (I)), and especially the compounds of formula (I) in which R more particularly represents an oleyl radical (unsaturated C₁₈ radical) or isostearyl (saturated C₁₈ radical), G denotes glucose, x is a value ranging from 1 to 2, especially isostearyl glucoside or oleyl glucoside, and mixtures thereof. This alkylpolyglucoside may be used as a mixture with a coemulsifier, more especially with a fatty alcohol and especially a fatty alcohol containing the same fatty chain as that of the alkylpolyglucoside, i.e. comprising from 14 to 24 carbon atoms and containing a branched and/or unsaturated chain, for example isostearyl alcohol when the alkylpolyglucoside is isostearyl glucoside, and oleyl alcohol when the alkylpolyglucoside is oleyl glucoside, optionally in the form of a self-emulsifying composition, as described, for example, in document WO-A-92/06778. Use may be made, for example, of the mixture of isostearyl glucoside and isostearyl alcohol, sold under the name Montanov WO 18 by the company SEPPIC, and also the mixture octyldodecanol and octyldodecyl xyloside sold under the name Fludanov 20X by the company SEPPIC.

Mention may also be made of succinic-terminated polyolefins, for instance esterified succinic-terminated polyisobutylenes and salts thereof, especially the diethanolamine salts, such as the commercial products sold under the names Lubrizol 2724, Lubrizol 2722 and Lubrizol 5603 by the company Lubrizol or the commercial product Chemcinnate 2000.

The total amount of emulsifiers in the composition will preferably be, in the composition for use in the invention, in active material contents ranging from 1% to 8% by weight and more particularly from 2% to 6% by weight relative to the total weight of the composition.

### FATTY PHASE

The compositions for use in the invention may contain at least one water-immiscible organic liquid phase, known as a fatty phase. This phase generally comprises one or more hydrophobic compounds that make the said phase water-immiscible. The said phase is liquid (in the absence of structuring agent) at room temperature (20-25°C). Preferentially, the water-immiscible organic-liquid organic phase in accordance with the invention generally comprises at least one volatile oil and/or non-volatile oil and optionally at least one structuring agent.

The term "oil" means a fatty substance that is liquid at room temperature (25°C) and atmospheric pressure (760 mmHg, i.e. 10⁶ Pa). The oil may be volatile or non-volatile.

For the purposes of the invention, the term "volatile oil" means an oil that is capable of evaporating on contact with the skin or the keratin fibre in less than one hour, at room temperature and atmospheric pressure. The volatile oils of the invention are volatile cosmetic oils, which are liquid at room temperature, having a non-zero vapour pressure, at room temperature and atmospheric pressure, ranging in particular from 0.13 Pa to 40 000 Pa (10⁻³ to 300 mmHg), in particular ranging from 1.3 Pa to 13 000 Pa (0.01 to 100 mmHg) and more particularly ranging from 1.3 Pa to 1300 Pa (0.01 to 10 mmHg).

The term "non-volatile oil" means an oil that remains on the skin or the keratin fibre at room temperature and atmospheric pressure for at least several hours, and that especially has a vapour pressure of less than 10⁻³ mmHg (0.13 Pa).

The oil may be chosen from any physiologically acceptable oil and in particular cosmetically acceptable oil, especially mineral, animal, plant or synthetic oils; in particular volatile or nonvolatile hydrocarbon-based oils and/or silicone oils and/or fluoro oils, and mixtures thereof.

More precisely, the term "hydrocarbon-based oil" means an oil mainly comprising carbon and hydrogen atoms and optionally one or more functions chosen from hydroxyl, ester, ether and carboxylic functions. Generally, the oil has a viscosity of from 0.5 to 100 000 mPa.s, preferably from 50 to 50 000 mPa.s and more preferably from 100 to 300 000 mPa.s.

As examples of volatile oils that may be used in the invention, mention may be made of:
- volatile hydrocarbon-based oils chosen from hydrocarbon-based oils containing from 8 to 16 carbon atoms, and especially C₈-C₁₆ isoalkanes of petroleum origin (also known as isoparaffins), for instance isododecane (also known as 2,2,4,4,6-pentamethylheptane), isodecane and isohexadecane, for example the oils sold under the trade names Isopar or Permethyl, branched C₈-C₁₆ esters and isohexyl neopentanoate, and mixtures thereof. Other volatile hydrocarbon-based oils, for instance petroleum distillates, especially those sold under the name Shell Solt by the company Shell, may also be used; volatile linear alkanes, such as those described in patent application DE10 2008 012 457 from the company Cognis.
- volatile silicones, for instance volatile linear or cyclic silicone oils, especially those with a viscosity ≤ 8 centistokes (8×10⁻⁶ m²/s) and especially containing from 2 to 7 silicon atoms, these silicones optionally comprising alkyl or alkoxy groups containing from 1 to 10 carbon atoms. As volatile silicone oils that may be used in the invention, mention may be made especially of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane;
- and mixtures thereof.

Mention may also be made of linear volatile alkyltrisiloxane oils of general formula (I): 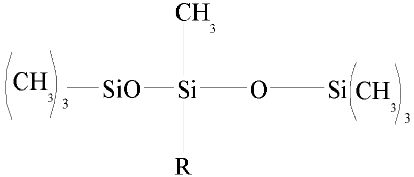 in which R represents an alkyl group containing from 2 to 4 carbon atoms, of which one or more hydrogen atoms may be substituted with a fluorine or chlorine atom.

Among the oils of general formula (I) that may be mentioned are:
3-butyl-1,1,1,3,5,5,5-heptamethyltrisiloxane,
3-propyl-1,1,1,3,5,5,5-heptamethyltrisiloxane, and
3-ethyl-1,1,1,3,5,5,5-heptamethyltrisiloxane,
corresponding to the oils of formula (I) for which R is, respectively, a butyl group, a propyl group or an ethyl group.

As examples of nonvolatile oils that may be used in the invention, mention may be made of:
- hydrocarbon-based oils of animal origin, such as perhydrosqualene;
- hydrocarbon-based plant oils such as liquid triglycerides of fatty acids of 4 to 24 carbon atoms, for instance heptanoic or octanoic acid triglycerides, or wheatgerm oil, olive oil, sweet almond oil, palm oil, rapeseed oil, cottonseed oil, alfalfa oil, poppy oil, pumpkin oil, marrow oil, blackcurrant oil, evening primrose oil, millet oil, barley oil, quinoa oil, rye oil, safflower oil, candlenut oil, passion flower oil, musk rose oil, sunflower oil, corn oil, soybean oil, grapeseed oil, sesame seed oil, hazelnut oil, apricot oil, macadamia oil, castor oil, avocado oil, caprylic/capric acid triglycerides, for instance those sold by the company Stéarineries Dubois or those sold under the names Miglyol 810, 812 and 818 by the company Dynamit Nobel, jojoba oil and shea butter oil,
- linear or branched hydrocarbons, of mineral or synthetic origin, such as liquid paraffins and derivatives thereof, petroleum jelly, polydecenes, polybutenes, hydrogenated polyisobutene such as Parleam, and squalane,
- synthetic ethers containing from 10 to 40 carbon atoms;
- synthetic esters, especially of fatty acids, for instance the oils of formula R₁COOR₂ in which R₁ represents a linear or branched higher fatty acid residue containing from 1 to 40 carbon atoms and R₂ represents a hydrocarbon-based chain, which is especially branched, containing from 1 to 40 carbon atoms, with R1 + R₂ ≥ 10, for instance purcellin oil (cetostearyl octanoate), isononyl isononanoate, isopropyl myristate, isopropyl palmitate, C₁₂-C₁₅ alkyl benzoates, hexyl laurate, diisopropyl adipate, isononyl isononanoate, 2-ethylhexyl palmitate, 2-octyldodecyl stearate, 2-octyldodecyl erucate, isostearyl isostearate or tridecyl trimellitate; alcohol or polyalcohol octanoates, decanoates or ricinoleates, for instance propylene glycol dioctanoate; hydroxylated esters, for instance isostearyl lactate, octyl hydroxystearate, octyldodecyl hydroxystearate, diisostearyl malate, triisocetyl citrate, and fatty alcohol heptanoates, octanoates or decanoates; polyol esters, for instance propylene glycol dioctanoate, neopentyl glycol diheptanoate or diethylene glycol diisononanoate; and pentaerythritol esters, for instance pentaerythrityl tetraisostearate,
- fatty alcohols that are liquid at room temperature, containing a branched and/or unsaturated carbon-based chain containing from 12 to 26 carbon atoms, for instance octyldodecanol, isostearyl alcohol, 2-butyloctanol, 2-hexyldecanol, 2-undecylpentadecanol or oleyl alcohol,
- higher fatty acids such as oleic acid, linoleic acid or linolenic acid;
- carbonates;
- acetates;
- citrates;
- fluoro oils that are optionally partially hydrocarbon-based and/or silicone-based, for instance fluorosilicone oils, fluoro polyethers and fluorosilicones as described in document EP-A-847 752;
- silicone oils, for instance linear or cyclic non-volatile polydimethylsiloxanes (PDMS); polydimethylsiloxanes comprising alkyl, alkoxy or phenyl groups, which are pendant or at the end of a silicone chain, these groups containing from 2 to 24 carbon atoms; phenyl silicones, for instance phenyl trimethicones, phenyl dimethicones, phenyl trimethylsiloxy diphenyl siloxanes, diphenyl dimethicones, diphenyl methyldiphenyl trisiloxanes and 2-phenylethyl trimethylsiloxy silicates, and
- mixtures thereof.

### STRUCTURING AGENT

The leave-in compositions for use in the invention comprising a fatty phase may also contain at least one agent for structuring the said fatty phase, which may preferably be chosen from waxes, pasty compounds, and mineral or organic lipophilic gelling agents, and mixtures thereof.

It is understood that the amount of these compounds may be adjusted by a person skilled in the art so as not to harm the desired effect in the context of the present invention.

### Wax(es)

The wax is in general a lipophilic compound that is solid at room temperature (25°C), with a solid/liquid reversible change of state, having a melting point of greater than or equal to 30°C, which may be up to 200°C and in particular up to 120°C.

In particular, the waxes that are suitable for the invention may have a melting point of greater than or equal to 45°C and in particular greater than or equal to 55°C.

For the purposes of the invention, the melting point corresponds to the temperature of the most endothermic peak observed on thermal analysis (DSC) as described in standard ISO 11357-3; 1999. The melting point of the wax may be measured using a differential scanning calorimeter (DSC), for example the calorimeter sold under the name MDSC 2920 by the company TA Instruments.

### The measuring protocol is as follows:

A sample of 5 mg of wax placed in a crucible is subjected to a first temperature rise ranging from -20°C to 100°C, at a heating rate of 10°C/minute, it is then cooled from 100°C to -20°C at a cooling rate of 10°C/minute and is finally subjected to a second temperature increase ranging from -20°C to 100°C at a heating rate of 5°C/minute. During the second temperature increase, the variation of the difference in power absorbed by the empty crucible and by the crucible containing the sample of wax is measured as a function of the temperature. The melting point of the compound is the temperature value corresponding to the top of the peak of the curve representing the variation in the difference in power absorbed as a function of the temperature.

The waxes that may be used in the compositions for use in the invention are chosen from waxes that are solid at room temperature of animal, plant, mineral or synthetic origin, and mixtures thereof.

As illustrations of waxes that are suitable for the invention, mention may be made especially of hydrocarbon-based waxes, for instance beeswax, lanolin wax, Chinese insect waxes, rice bran wax, carnauba wax, candelilla wax, ouricury wax, esparto grass wax, berry wax, shellac wax, Japan wax and sumach wax; montan wax, orange wax and lemon wax, refined sunflower wax sold under the name Sunflower Wax by Koster Keunen, microcrystalline waxes, paraffins and ozokerite; polyethylene waxes, the waxes obtained by Fischer-Tropsch synthesis and waxy copolymers, and also esters thereof.

Mention may also be made of waxes obtained by catalytic hydrogenation of animal or plant oils containing linear or branched C₈-C₃₂ fatty chains. Among these waxes that may especially be mentioned are isomerized jojoba oil such as the trans-isomerized partially hydrogenated jojoba oil manufactured or sold by the company Desert Whale under the commercial reference Iso-Jojoba-50®, hydrogenated sunflower oil, hydrogenated castor oil, hydrogenated coconut oil, hydrogenated lanolin oil and bis(1,1,1-trimethylolpropane) tetrastearate sold under the name Hest 2T-4S® by the company Heterene.

Mention may also be made of silicone waxes (C₃₀₋₄₅ alkyl dimethicone) and fluoro waxes.

The waxes obtained by hydrogenation of castor oil esterified with cetyl alcohol, sold under the names Phytowax ricin 16L64® and 22L73® by the company Sophim, may also be used. Such waxes are described in patent application FR-A-2 792 190.

A wax that may be used is a C₂₀-C₄₀ alkyl (hydroxystearyloxy)stearate (the alkyl group containing from 20 to 40 carbon atoms), alone or as a mixture.

Such a wax is especially sold under the names Kester Wax K 82 P®, Hydroxypolyester K 82 P® and Kester Wax K 80 P® by the company Koster Keunen.

As microwaxes that may be used in the compositions for use in the invention, mention may be made especially of carnauba microwaxes, such as the product sold under the name MicroCare 350® by the company Micro Powders, synthetic microwaxes, such as the product sold under the name MicroEase 114S® by the company Micro Powders, microwaxes consisting of a mixture of carnauba wax and polyethylene wax, such as the products sold under the names Micro Care 300® and 310® by the company Micro Powders, microwaxes consisting of a mixture of carnauba wax and of synthetic wax, such as the product sold under the name Micro Care 325® by the company Micro Powders, polyethylene microwaxes, such as the products sold under the names Micropoly 200®, 220®, 220L® and 250S® by the company Micro Powders, the commercial products Performalene 400, Polyethylene and Performalene 500-L Polyethylene from New Phase Technologies, Performalene 655, Polyethylene or paraffin waxes, for instance the wax having the INCI name Microcrystalline Wax and Synthetic Wax and sold under the trade name Microlease by the company Sochibo; polytetrafluoroethylene microwaxes such as those sold under the names Microslip 519® and 519 L® by the company Micro Powders.

The composition for use in the invention may preferably comprise a content of wax(es) ranging from 3% to 20% by weight relative to the total weight of the composition, in particular from 5% to 15% and more particularly from 6% to 15% thereof.

According to one particular form of the invention, in the context of anhydrous solid compositions in stick form, use will be made of polyethylene microwaxes in the form of crystallites with an aspect ratio at least equal to 2, and with a melting point ranging from 70 to 110°C and preferably from 70 to 100°C, so as to reduce or even eliminate the presence of strata in the solid composition.

These crystallites in needle form and especially the dimensions thereof may be characterized visually according to the following method.

The wax is deposited on a microscope slide, which is placed on a hotplate. The slide and the wax are heated to a temperature generally at least 5°C higher than the melting point of the wax or of the mixture of waxes under consideration. At the end of melting, the liquid thus obtained and the microscope slide are allowed to cool to solidify. Observation of the crystallites is performed using a Leica DMLB100 optical microscope, with an objective lens selected as a function of the size of the objects to be viewed, and under polarized light. The dimensions of the crystallites are measured using image analysis software such as that sold by the company Microvision.

The crystallite polyethylene waxes in accordance with the invention preferably have an average length ranging from 5 to 10 µm. The term "average length" denotes the dimension given by statistical granulometric distribution of half the population, which is written as D50.

Use will be made more particularly of a mixture of the waxes Performalene 400 Polyethylene and Performalene 500-L Polyethylene from New Phase Technologies.

### Pasty compounds

For the purposes of the present invention, the term "pasty compound" is intended to denote a lipophilic fatty compound that undergoes a reversible solid/liquid change of state and that comprises, at a temperature of 23°C, a liquid fraction and a solid fraction.

The pasty compound is preferably chosen from synthetic compounds and compounds of plant origin. A pasty compound may be obtained by the synthesis from starting materials of plant origin.

The pasty compound may be advantageously chosen from:
- lanolin and derivatives thereof,
- polymeric or non-polymeric silicone compounds,
- polymeric or non-polymeric fluoro compounds,
- vinyl polymers, especially:
- olefin homopolymers,
- olefin copolymers,
- hydrogenated diene homopolymers and copolymers,
- linear or branched oligomers, homopolymers or copolymers of alkyl (meth)acrylates preferably containing a C₈-C₃₀ alkyl group,
- oligomers, homopolymers and copolymers of vinyl esters containing C₈-C₃₀ alkyl groups,
- oligomers, homopolymers and copolymers of vinyl ethers containing C₈-C₃₀ alkyl groups,
- liposoluble polyethers resulting from the polyetherification between one or more C₂-C₁₀₀ and preferably C₂-C₅₀ diols,
- esters,
- mixtures thereof.

Among the esters, the following are especially preferred:
- esters of a glycerol oligomer, especially diglycerol esters, in particular condensates of adipic acid and of glycerol, for which some of the hydroxyl groups of the glycerols have reacted with a mixture of fatty acids such as stearic acid, capric acid, stearic acid and isostearic acid, and 12-hydroxystearic acid, especially such as those sold under the brand name Softisan 649 by the company Sasol,
- the arachidyl propionate sold under the brand name Waxenol 801 by Alzo,
- phytosterol esters,
- fatty acid triglycerides and derivatives thereof,
- pentaerythritol esters,
- non-crosslinked polyesters resulting from polycondensation between a linear or branched C₄-C₅₀ dicarboxylic acid or polycarboxylic acid and a C₂-C₅₀ diol or polyol,
- aliphatic esters of an ester, resulting from the esterification of an aliphatic hydroxycarboxylic acid ester with an aliphatic carboxylic acid,
- polyesters resulting from the esterification, with a polycarboxylic acid, of an aliphatic hydroxycarboxylic acid ester, the said ester comprising at least two hydroxyl groups, such as the products Risocast DA-H® and Risocast DA-L®,
- esters of a diol dimer and of a diacid dimer, where appropriate esterified on their free alcohol or acid function(s) with acid or alcohol radicals, such as Plandool-G,
- mixtures thereof.

Among the pasty compounds of plant origin that will preferably be chosen is a mixture of oxyethylenated (5 OE) oxypropylenated (5 OP) soybean sterols and pentaerythritol, sold under the reference Lanolide by the company Vevy.

### Lipophilic gelling agents

### Mineral gelling agents

Mineral lipophilic gelling agents that may be mentioned include optionally modified clays, for instance hectorites modified with a C10-C22 ammonium chloride, for instance hectorite modified with distearyldimethylammonium chloride, for instance the product sold under the name Bentone 38V® by the company Elementis.

Mention may also be made of fumed silica optionally subjected to a hydrophobic surface treatment, the particle size of which is less than 1 µm. Specifically, it is possible to chemically modify the surface of the silica, by chemical reaction generating a reduced number of silanol groups present at the surface of the silica. It is especially possible to substitute silanol groups with hydrophobic groups: a hydrophobic silica is then obtained. The hydrophobic groups may be trimethylsiloxyl groups, which are obtained especially by treating fumed silica in the presence of hexamethyldisilazane. Silicas thus treated are known as "silica silylate" according to the CTFA (8th Edition, 2000). They are sold, for example, under the references Aerosil R812® by the company Degussa, Cab-O-Sil TS-530® by the company Cabot, dimethylsilyloxyl or polydimethylsiloxane groups, which are obtained especially by treating fumed silica in the presence of polydimethylsiloxane or dimethyldichlorosilane. Silicas thus treated are known as "silica dimethyl silylate" according to the CTFA (8th Edition, 2000). They are sold, for example, under the references Aerosil R972® and Aerosil R974® by the company Degussa, and Cab-O-Sil TS-610® and Cab-O-Sil TS-720® by the company Cabot.

The hydrophobic fumed silica preferably has a particle size that may be nanometric to micrometric, for example ranging from about 5 to 200 nm.

### Organic gelling agents

The polymeric organic lipophilic gelling agents are, for example, partially or totally crosslinked elastomeric organopolysiloxanes of three-dimensional structure, for instance those sold under the names KSG6®, KSG16® and KSG18® from Shin-Etsu, Trefil E-505C® or Trefil E-506C® from Dow Corning, Gransil SR-CYC®, SR DMF10®, SR-DC556®, SR 5CYC gel®, SR DMF 10 gel® and SR DC 556 gel® from Grant Industries and SF 1204® and JK 1130 from General Electric; ethylcellulose, for instance the product sold under the name Ethocel® by Dow Chemical; galactomannans comprising from one to six and in particular from two to four hydroxyl groups per saccharide, substituted with a saturated or unsaturated alkyl chain, for instance guar gum alkylated with C1 to C6, and in particular C1 to C3, alkyl chains, and mixtures thereof. Block copolymers of "diblock", "triblock" or "radial" type, of the polystyrene/polyisoprene or polystyrene/polybutadiene type, such as the products sold under the name Luvitol HSB® by the company BASF, of the polystyrene/copoly(ethylene-propylene) type, such as the products sold under the name Kraton® by the company Shell Chemical Co., or of the polystyrene/copoly(ethylene-butylene) type, and mixtures of triblock and radial (star) copolymers in isododecane, such as those sold by the company Penreco under the name Versagel®, for instance the mixture of butylene/ethylene/styrene triblock copolymer and of ethylene/propylene/styrene star copolymer in isododecane (Versagel M 5960).

Lipophilic gelling agents that may also be mentioned include polymers with a weight-average molecular mass of less than 100 000, comprising a) a polymer backbone with hydrocarbon-based repeating units containing at least one heteroatom, and optionally b) at least one optionally functionalized pendent fatty chain and/or terminal fatty chain, containing from 6 to 120 carbon atoms and being linked to these hydrocarbon-based units, as described in patent applications WO-A-02/056 847 and WO-A-02/4/619, in particular, polyamide resins (especially comprising alkyl groups containing from 12 to 22 carbon atoms) such as those described in US-A-5 783 657,

Among the lipophilic gelling agents that may be used in the compositions for use in the invention, mention may also be made of fatty acid esters of dextrin, such as dextrin palmitates, especially the products sold under the name Rheopearl TL® or Rheopearl KL® by the company Chiba Flour.

Silicone polyamides of the polyorganosiloxane type may also be used, such as those described in documents US-A-5 874 069, US-A-5 919 441, US-A-6 051 216 and US-A-5 981 680.

These silicone polymers may belong to the following two families:
- polyorganosiloxanes comprising at least two groups capable of establishing hydrogen interactions, these two groups being in the chain of the polymer, and/or
- polyorganosiloxanes comprising at least two groups capable of establishing hydrogen interactions, these two groups being located on grafts or branches.

### ADDITIONAL ANTIPERSPIRANT SALTS OR COMPLEXES

According to one particular form of the invention, the compositions for use in the invention may also contain an additional antiperspirant salt or complex.

The term "agent for treating perspiration" means any substance which, by itself, has the effect of reducing the sensation on the skin of moisture associated with human sweat, or of masking human sweat.

The additional antiperspirant salts or complexes in accordance with the invention are generally chosen from aluminium and/or zirconium salts or complexes. They are preferably chosen from aluminium halohydrates; aluminium zirconium halohydrates, complexes of zirconium hydroxychloride and of aluminium hydroxychloride with or without an amino acid, such as those described in patent US-3 792 068.

Among the aluminium salts, mention may be made in particular of aluminium chlorohydrate in activated or unactivated form, aluminium chlorohydrex, the aluminium chlorohydrex-polyethylene glycol complex, the aluminium chlorohydrex-propylene glycol complex, aluminium dichlorohydrate, the aluminium dichlorohydrex-polyethylene glycol complex, the aluminium dichlorohydrex-propylene glycol complex, aluminium sesquichlorohydrate, the aluminium sesquichlorohydrex-polyethylene glycol complex, the aluminium sesquichlorohydrex-propylene glycol complex, aluminium sulfate buffered with sodium aluminium lactate.

Among the aluminium-zirconium salts, mention may be made in particular of aluminium zirconium octachlorohydrate, aluminium zirconium pentachlorohydrate, aluminium zirconium tetrachlorohydrate and aluminium zirconium trichlorohydrate.

The complexes of zirconium hydroxychloride and of aluminium hydroxychloride with an amino acid are generally known as ZAG (when the amino acid is glycine).

Among these products, mention may be made of the complexes aluminium zirconium octachlorohydrex glycine, aluminium zirconium pentachlorohydrex glycine, aluminium zirconium tetrachlorohydrex glycine and aluminium zirconium trichlorohydrex glycine.

The antiperspirant salts or complexes may be present in the composition for use in the invention in a proportion from about 0.5% to 25% by weight relative to the total weight of the composition.

### DEODORANT ACTIVE AGENTS

The compositions for use in the invention may also contain one or more additional deodorant active agents.

The term "deodorant active agent" refers to any substance that is capable of masking, absorbing, improving and/or reducing the unpleasant odour resulting from the decomposition of human sweat by bacteria.

The deodorant active agents may be bacteriostatic agents or bactericides that act on underarm odour microorganisms, such as 2,4,4'-trichloro-2'-hydroxydiphenyl ether (®Triclosan), 2,4-dichloro-2'-hydroxydiphenyl ether, 3',4',5'-trichlorosalicylanilide, 1-(3',4'-dichlorophenyl)-3-(4'-chlorophenyl)urea (®Triclocarban) or 3,7,11-trimethyldodeca-2,5,10-trienol (®Farnesol); quaternary ammonium salts such as cetyltrimethylammonium salts, cetylpyridinium salts, DPTA (1,3-diaminopropanetetraacetic acid), 1,2-decanediol (Symclariol from the company Symrise), glycerol derivatives, for instance caprylic/capric glycerides (Capmul MCM from Abitec), glyceryl caprylate or caprate (Dermosoft GMCY and Dermosoft GMC, respectively from Straetmans), Polyglyceryl-2 caprate (Dermosoft DGMC from Straetmans), and biguanide derivatives, for instance polyhexamethylene biguanide salts. - chlorhexidine and salts thereof; 4-phenyl-4,4-dimethyl-2-butanol (Symdeo MPP from Symrise).

Among the deodorant active agents in accordance with the invention, mention may also be made of - zinc salts, for instance zinc salicylate, zinc gluconate, zinc pidolate; zinc sulfate, zinc chloride, zinc lactate, zinc phenolsulfonate; zinc ricinoleate;
- sodium bicarbonate;
- salicylic acid and derivatives thereof such as 5-n-octanoylsalicylic acid;
- silver zeolites or silver-free zeolites;
- alum.

The deodorant active agents may be present in the composition for use in the invention in a proportion from about 0.01% to 5% by weight relative to the total weight of the composition.

### SUSPENSION AGENTS

In order to improve the homogeneity of the product, it is also possible to use one or more suspension agents preferably chosen from hydrophobic modified montmorillonite clays such as hydrophobic modified bentonites or hectorites.

Examples that may be mentioned include the product Stearalkonium Bentonite (CTFA name) (product of reaction of bentonite and the quaternary ammonium stearalkonium chloride) such as the commercial product sold under the name Tixogel MP 250 by the company Sud Chemie Rheologicals, United Catalysts Inc. or the product Disteardimonium Hectorite (CTFA name) (product of reaction of hectorite and distearyldimonium chloride) sold under the name Bentone 38 or Bentone Gel by the company Elementis Specialities.

The suspension agents are preferably present in amounts ranging from 0.1% to 5% by weight and more preferentially from 0.2% to 2% by weight relative to the total weight of the composition.

### ORGANIC POWDER

According to one particular form of the invention, the compositions for use in the invention will also contain an organic powder.

In the present patent application, the term "organic powder" means any solid that is insoluble in the medium at room temperature (25°C).

As organic powders that may be used in the composition for use in the invention, examples that may be mentioned include polyamide particles and especially those sold under the name Orgasol by the company Atochem; polyethylene powders; microspheres based on acrylic copolymers, such as those made of ethylene glycol dimethacrylate/lauryl methacrylate copolymer, sold by the company Dow Corning under the name Polytrap; polymethyl methacrylate microspheres, sold under the name Microsphere M-100 by the Company Matsumoto or under the name Covabead LH85 by the Company Wackherr; hollow polymethyl methacrylate microspheres (particle size: 6.5-10.5 µm) sold under the name Ganzpearl GMP 0800 by Ganz Chemical; methyl methacrylate/ethylene glycol dimethacrylate copolymer microbeads (size: 6.5-10.5 µm) sold under the name Ganzpearl GMP 0820 by Ganz Chemical or Microsponge 5640 by the company Amcol Health & Beauty Solutions; ethylene-acrylate copolymer powders, such as those sold under the name Flobeads by the company Sumitomo Seika Chemicals; expanded powders such as hollow microspheres and especially microspheres formed from a terpolymer of vinylidene chloride, acrylonitrile and methacrylate and sold under the name Expancel by the company Kemanord Plast under the references 551 DE 12 (particle size of about 12 µm and mass per unit volume of 40 kg/m³), 551 DE 20 (particle size of about 30 µm and mass per unit volume of 65 kg/m³), 551 DE 50 (particle size of about 40 µm), or the microspheres sold under the name Micropearl F 80 ED by the company Matsumoto; powders of natural organic materials such as starch powders, especially of crosslinked or non-crosslinked corn, wheat or rice starch, such as the powders of starch crosslinked with octenylsuccinic anhydride, sold under the name Dry-Flo by the company National Starch; silicone resin microbeads such as those sold under the name Tospearl by the company Toshiba Silicone, especially Tospearl 240; amino acid powders such as the lauroyllysine powder sold under the name Amihope LL-11 by the company Ajinomoto; particles of wax microdispersion, which preferably have mean sizes of less than 1 µm and especially ranging from 0.02 µm to 1 µm, and which are formed essentially from a wax or a mixture of waxes, such as the products sold under the name Aquacer by the company Byk Cera, and especially: Aquacer 520 (mixture of synthetic and natural waxes), Aquacer 514 or 513 (polyethylene wax), Aquacer 511 (polymeric wax), or such as the products sold under the name Jonwax 120 by the company Johnson Polymer (mixture of polyethylene wax and paraffin wax) and under the name Ceraflour 961 by the company Byk Cera (micronized modified polyethylene wax); and mixtures thereof.

### ADDITIVES

The cosmetic compositions for use in the invention may also comprise cosmetic adjuvants chosen from softeners, antioxidants, opacifiers, stabilizers, moisturizers, vitamins, bactericides, preserving agents, polymers, fragrances, thickeners, propellants or any other ingredient usually used in cosmetics for this type of application.

Needless to say, a person skilled in the art will take care to select this or these optional additional compounds such that the advantageous properties intrinsically associated with the cosmetic composition in accordance with the invention are not, or are not substantially, adversely affected by the envisaged addition(s).

The thickeners, which are preferably nonionic, may be chosen from modified or unmodified guar gums and celluloses such as hydroxypropyl guar gum, cetylhydroxyethylcellulose, silicas, for instance Bentone Gel MIO sold by the company NL Industries or Veegum Ultra sold by the company Polyplastic.

The thickeners may also be cationic, for instance Polyquaternium-37 sold under the name Salcare SC95 (Polyquaternium-37 (and) Mineral Oil (and) PPG-1 Trideceth-6) or Salcare SC96 (Polyquaternium-37 (and) Propylene Glycol Dicaprylate/Dicaprate (and) PPG-1 Trideceth-6) or other crosslinked cationic polymers, for instance those of the CTFA name Ethyl Acrylate/Dimethylaminoethyl Methacrylate Cationic Copolymer In Emulsion.

The amounts of these various constituents that may be present in the cosmetic composition for use in the invention are those conventionally used in compositions for treating perspiration.

### AEROSOLS

The compositions for use in the invention may also be pressurized and may be conditioned in an aerosol device formed by:
(A) a container comprising an antiperspirant composition as defined previously,
(B) at least one propellant and a means for dispensing the said aerosol composition.

The propellants generally used in products of this type and that are well known to those skilled in the art are, for instance, dimethyl ether (DME); volatile hydrocarbons such as n-butane, propane, isobutane and mixtures thereof, optionally with at least one chlorohydrocarbon and/or fluorohydrocarbon; among these derivatives, mention may be made of the compounds sold by the company DuPont de Nemours under the names Freon® and Dymel®, and in particular monofluorotrichloromethane, difluorodichloromethane, tetrafluorodichloroethane and 1,1-difluoroethane sold especially under the trade name Dymel 152 A by the company DuPont. Carbon dioxide, nitrous oxide, nitrogen or compressed air may also be used as propellant.

The compositions containing perlite particles as defined previously and the propellant(s) may be in the same compartment or in different compartments in the aerosol container. According to the invention, the concentration of propellant generally ranges from 5% to 95% by weight of pressurized composition, and more preferentially from 50% to 85% by weight relative to the total weight of the pressurized composition.

The dispensing means, which forms a part of the aerosol device, is generally formed by a dispensing valve controlled by a dispensing head, which itself comprises a nozzle via which the aerosol composition is vaporized. The container containing the pressurized composition may be opaque or transparent. It may be made of glass, a polymer or a metal, optionally coated with a protective varnish coat.

The examples that follow serve to illustrate the present invention. The amounts are given as mass percentages relative to the total weight of the composition.

### EXAMPLES

### Example 1: Antiperspirant roll-on

| Ingredients | Amounts in weight % |
|---|---|
| Dihydroxyacetone (Vegetan Dihydroxyacetone Cristal (Soliance) | 5% |
| Ethanol | 44.5% |
| Water | qs 100% |

The antiperspirant efficacy according to Example 1 was evaluated on panel of 22 women, according to the following protocol:
i) Two times 8 areas (4 × 5 cm²) are delimited on either side of the spinal column. Each product area has a corresponding symmetrical untreated control area.
ii) Antiperspirant products are applied for 4 days, by light massaging. Total amount applied: 3.75 mg/cm²
iii) Occlusion for 1 hour.

### 24 hours after the last application

iv) The back is washed with water to remove any remaining trace of product; cellulose squares are fixed onto the various areas, and the person is then made to sweat in a sauna for 15 minutes at 80°C.
v) The amount of sweat is evaluated by weighing the cellulose squares before and after sweating.

Under the conditions of the test, the reduction of sweating after treatment with the roll-on containing DHA is 43% ± 12%.

### Example 2: Antiperspirant roll-or (not according to the invention)

| Ingredients | Amounts in weight % |
|---|---|
| Ribulose | 10% |
| PEG-136/Steareth-100/HDI (Rheolate) | 2% |
| Mixture of C₁₄-C₂₂ alcohol/C₁₂-C₂₀ alkyl polyglucoside (Montanov L - SEPPIC) | 5% |
| Dimethicone 200 cSt (Dow Corning 200 Fluid - Dow Corning) | 7% |
| Preserving agents | 0.15% |
| Water | qs 100% |

### Example 3: Antiperspirant stick

| | Ingredients | Amounts in weight % |
|---|---|---|
| A. | Polyethylene (Performalene 400) | 9% |
| | Beheneth-10 (Emulgin BA 10) | 2% |
| | Cetyl PEG/PPG-10/1 Dimethicone (ABIL EM 90 - Degussa) | 5.25% |
| | Isopropyl palmitate | 9% |
| | Cyclopentasiloxane (Dow Corning 245 Fluid) | 6% |
| B1 | Preserving agents | 0.3% |
| | DHA | 2.5% |
| | Water | qs 100% |

Phase A is introduced into phase B. The mixture is heated at 90°C until homogenized, and the sticks are then cast at 91 °C.

## Claims

1. Cosmetic use, as an agent for treating perspiration, of at least one compound comprising at least one free carbonyl function that is capable of reacting with at least one amine compound via the Maillard reaction, said at least one compound comprising at least one free carbonyl function being a carbohydrate and/or a precursor thereof and/or an oxidized form thereof, and in which the carbohydrate is dihydroxyacetone (DHA) in free form and/or in encapsulated form.

2. Use according to Claim 1, in which the carbohydrate is an ester precursor of dihydroxyacetone such as a dihydroxyacetone phosphate.

3. Use according to any one of Claims 1 or 2, in which the compound comprising at least one free carbonyl function is combined with amine compounds, for instance amino acids such as lysine or proline, or with agents that are capable of modulating the conformation of proteins, for instance chaotropic agents (for example urea or urea derivatives, guanidine salts), cosmotropic agents (sulfates, phosphates, lithium, magnesium, tert-butanol), surfactants, dehydrating agents or protease inhibitors.

4. Use according to any one of Claims 1 to 3, in which the compound(s) comprising at least one free carbonyl function that is capable of reacting with at least one amine compound via the Maillard reaction is present in a composition in a concentration ranging from 0.1% to 20% by weight and preferably from 1% to 10% by weight relative to the total weight of the composition.

5. Use according to Claim 4, **characterized in that** the composition is in the form of aqueous gels, aqueous or aqueous-alcoholic solutions, simple or multiple emulsions, vesicular dispersions of ionic and/or nonionic type, or wax/aqueous phase dispersions.

6. Use according to any one of Claims 4 to 5, **characterized in that** the composition is anhydrous.

7. Use according to any one of Claims 4 to 5, **characterized in that** the composition comprises an aqueous phase and more preferentially comprises at least 20% by weight of water relative to the total weight of the composition.

## Patentansprüche

1. Kosmetische Verwendung von mindestens einer Verbindung, die mindestens eine freie Carbonylfunktion, die über die Maillard-Reaktion zur Reaktion mit mindestens einer Aminverbindung in der Lage ist, umfasst, als ein Mittel zur Behandlung von Transpiration, wobei die mindestens eine mindestens eine freie Carbonylfunktion umfassende Verbindung ein Kohlenhydrat und/oder ein Vorläufer davon und/oder eine oxidierte Form davon ist, und bei der das Kohlenhydrat Dihydroxyaceton (DHA) in freier Form und/oder in verkapselter Form ist.

2. Verwendung nach Anspruch 1, bei der das Kohlenhydrat ein Estervorläufer von Dihydroxyaceton wie Dihydroxyacetonphosphat ist.

3. Verwendung nach einem der Ansprüche 1 oder 2, bei der die mindestens eine freie Carbonylfunktion umfassende Verbindung mit Aminverbindungen, beispielsweise Aminosäuren wie Lysin oder Prolin, oder mit Mitteln, die zur Modulation der Proteinkonformation, beispielsweise chaotrope Mittel (zum Beispiel Harnstoff oder Harnstoffderivate, Guanidinsalze), kosmotrope Mittel (Sulfate, Phosphate, Lithium, Magnesium, tert-Butanol), Tenside, Wasser entziehende Mittel oder Proteasehemmer, in der Lage sind, kombiniert wird.

4. Verwendung nach einem der Ansprüche 1 bis 3, bei der die Verbindung(en), die mindestens eine freie Carbonylfunktion, die über die Maillard-Reaktion zur Reaktion mit mindestens einer Aminverbindung in der Lage ist, umfasst bzw. umfassen, in einer Zusammensetzung in einer Konzentration vorliegt, die 0,1 Gewichts-% bis 20 Gewichts-% und vorzugsweise 1 Gewichts-% bis 10 Gewichts-%, bezogen auf das Gesamtgewicht der Zusammensetzung, beträgt.

5. Verwendung nach Anspruch 4, **dadurch gekennzeichnet, dass** die Zusammensetzung in der Form von wässrigen Gelen, wässrigen oder wässrig-alkoholischen Lösungen, Einfach- oder Mehrfachemulsionen, Blasendispersionen vom ionischen und/oder nichtionischen Typ oder Wachs/Wasserphase-Dispersionen ist.

6. Verwendung nach einem der Ansprüche 4 bis 5, **dadurch gekennzeichnet, dass** die Zusammensetzung wasserfrei ist.

7. Verwendung nach einem der Ansprüche 4 bis 5, **dadurch gekennzeichnet, dass** die Zusammensetzung eine wässrige Phase umfasst und weiter bevorzugt mindestens 20 Gewichts-% Wasser, bezogen auf das Gesamtgewicht der Zusammensetzung, umfasst.

## Revendications

1. Utilisation cosmétique, en tant qu'un agent pour le traitement de la transpiration, d'au moins un composé comprenant au moins une fonction carbonyle libre qui est capable de réagir avec au moins un composé de type amine via la réaction de Maillard, ledit au moins un composé comprenant au moins une fonction carbonyle libre étant un glucide et/ou un précurseur correspondant et/ou une forme oxydée correspondante, et dans laquelle le glucide est la dihydroxyacétone (DHA) sous forme libre et/ou sous forme encapsulée.

2. Utilisation selon la revendication 1, dans laquelle le glucide est un précurseur de dihydroxyacétone de type ester tel qu'un phosphate de dihydroxyacétone.

3. Utilisation selon l'une quelconque des revendications 1 et 2, dans laquelle le composé comprenant au moins une fonction carbonyle libre est combiné avec des composés de type amine, par exemple des acides aminés tels que la lysine ou la proline, ou avec des agents qui sont capables de moduler la conformation de protéines, par exemple des agents chaotropiques (par exemple l'urée ou des dérivés d'urée, des sels de guanidine), des agents cosmotropiques (sulfates, phosphates, lithium, magnésium, tert-butanol), des tensioactifs, des agents déshydratants ou des inhibiteurs de protéase.

4. Utilisation selon l'une quelconque des revendications 1 à 3, dans laquelle le(s) composé(s) comprenant au moins une fonction carbonyle libre qui est capable de réagir avec au moins un composé de type amine via la réaction de Maillard est/sont présent(s) dans une composition en une concentration dans la plage de 0,1 % à 20 % en poids et préférablement de 1 % à 10 % en poids par rapport au poids total de la composition.

5. Utilisation selon la revendication 4, **caractérisée en ce que** la composition est sous la forme de gels aqueux, de solutions aqueuses ou aqueuses-alcooliques, d'émulsions simples ou multiples, de dispersions vésiculaires de type ionique et/ou non ionique, ou de dispersions en phase cire/aqueuse.

6. Utilisation selon l'une quelconque des revendications 4 et 5, **caractérisée en ce que** la composition est anhydre.

7. Utilisation selon l'une quelconque des revendications 4 et 5, **caractérisée en ce que** la composition comprend une phase aqueuse et plus préférentiellement comprend au moins 20 % en poids d'eau par rapport au poids total de la composition.
